# EUROPEAN PATENT SPECIFICATION

(11) **EP 1 523 940 B1**
(45) Date of publication and mention of the grant of the patent: **17.08.2016**
(21) Application number: 04024291.9
(22) Date of filing: 12.10.2004
(51) Int. Cl.: A61B 8/14

(54) **Ultrasound diagnosis apparatus**
Ultraschalldiagnosegerät
Dispositif ultrasonore de diagnostic

(30) Priority: 14.10.2003 JP 2003354125
(43) Date of publication of application: 20.04.2005
(73) Proprietor: Hitachi Aloka Medical, Ltd., Mitaka-shi Tokyo 181-8622 (JP)
(72) Inventor: Ohtake, Akifumi, Mitaka-shi Tokyo 181-8622 (JP)
(74) Representative: Heim, Hans-Karl

(56) References cited:
- US-A- 4 390 025
- US-B1- 6 607 488

## Description

### BACKGROUND OF THE INVENTION

### 1. Field of the Invention

The present invention relates to an ultrasound diagnosis apparatus and in particular to display of a body mark and a probe mark.

### 2. Description of the Related Art

An ultrasound diagnosis apparatus has a function to display, on a screen of a display device, a "body mark (body symbol)" and a "probe mark (probe symbol)" as reference images along with an ultrasound image (image of living tissue, or a living body image). The body mark is typically a simple, two-dimensional figure schematically representing a partial shape within a living body. A user may operate the device to select a specific body mark corresponding to a body part to be diagnosed using ultrasound from among a plurality of body marks which are prepared in advance. The body mark is displayed near the living body image. In order to identify a position and direction of the probe during ultrasound diagnosis, a probe mark is displayed overlapping the body mark. The probe mark is typically a figure of a simple line or simple box. The user can freely set the position and direction of the probe mark on the body mark. These marks are important information for identifying the part for which the living body image is obtained, on the display screen or in an examination report.

In the related art, when a body mark and a probe mark are to be displayed along with currently obtained living body image, user operations such as mark selection and mark positioning are necessary. In the related art, it is possible to replay and display a static image or animation image by reading received data from a cine-memory storing the received data. In this configuration also, it is necessary that user operations such as mark selection and mark positioning be repeated. These operations are complicated for the users. Moreover, there is a disadvantage that it is difficult to appropriately set the probe mark.

Japanese Patent Laid-Open Publication No. 2000-201926 discloses an apparatus in which a three-dimensional body mark and a three-dimensional probe mark are displayed. In this apparatus, when a user changes a position of a probe mark, display content of a body mark is automatically changed so that the position of the probe mark is at a center position of the body mark. Japanese Patent Laid-Open Publication No. 2001-017433 also discloses an apparatus in which a three-dimensional body mark and a three-dimensional probe mark are displayed. In this apparatus, a body mark and a probe mark seen from a viewing direction designated by the user using an input unit are generated. A probe mark is displayed on an appropriate position on a body mark based on an actual positional relationship between a living body and the probe. In this case, the actual positional relationship is measured using a magnetic sensor (refer to paragraph 0025 of Japanese Patent Laid-Open Publication No. 2001-017433).

None of the references, however, discloses a technique for automatically displaying the body mark and the probe mark when received data is replayed. Moreover, none of the references discloses automatic determination of the orientation of the probe mark in addition to the position of the probe mark.

US 4,390,025 teaches an ultrasonic diagnostic apparatus including an apparatus for detecting the position of an ultrasonic probe. It is provided a memory for storing the position of the ultrasonic probe and a memory for storing thomogram signals received by the ultrasonic probe. Furtheron, it is used a timing pulse generator to synchronize the data stored in the two memories. Then after the buffering of the data of these two memories, the data is mixed and displayed on a monitor.

A method and a system for indicating the position and orientation of a scanned plan relative to a patient is disclosed in US 6,607,488 B1. According to this system, an image which is presented on a display is oriented based on the original orientation of the ultrasonic probe.

### SUMMARY OF THE INVENTION

The present invention advantageously provides an ultrasound diagnosis apparatus in which a reference image representing a measurement condition when received data is obtained can be automatically generated during replay of the received data.

The present invention advantageously provides an ultrasound diagnosis apparatus in which the burden on the user can be reduced during display of a body mark and a probe mark.

The present invention advantageously provides an ultrasound diagnosis apparatus in which a body mark and a probe mark accurately reflecting actual measurement conditions can be displayed.
(1) According to one aspect of the present invention, there is provided an ultrasound diagnosis apparatus according to claim 1.

With this configuration, coordinate data which represents at least one of the spatial position and orientation of the probe is obtained. The coordinate data is preferably data which represents both the spatial position of the probe and the orientation of the probe. When the received data is stored, coordinate data to be correlated to the received data is also stored. When the received data is read, corresponding coordinate data correlated to the received data is also read. When a living body image is replayed and displayed, a reference image generated based on the coordinate data is displayed along with the living body image. By observing the reference image, it is possible to easily identify contents of the coordinate data, that is, measurement conditions when the received data is obtained (for example, probe position and probe orientation with respect to the living body). In this manner, because a reference image can be displayed during replay of the received data without a complicated setting, it is possible to reduce or remove the burden on the user, and to accurately know the measurement conditions.

In the above-described configuration, the probe may be, for example, a probe for measuring two-dimensional data or a probe for measuring three-dimensional data. As the coordinate measuring unit, it is preferable to use a magnetic field measurement system as will be described below. Alternatively, it is also possible to use, as the coordinate measuring unit, a mechanical measurement system, an optical measurement system, a measurement system which uses electric waves, or a measurement system which uses ultrasound, for example. The living body image is preferably a two-dimensional or three-dimensional ultrasound image. The reference image is preferably a two-dimensional or three-dimensional graphical image. The reference image may be a digital photographic image. The living body image and the reference image are preferably displayed on a single screen. Alternatively, it is also possible to display one of the living body image and the reference image on a main display and the other image on an auxiliary display. The received data is typically managed in units of beams, frames, or volumes. In general, one set of coordinate data is correlated to one set of received data. Alternatively, it is also possible to correlate one set of coordinate data to a plurality of received data sets, or to correlate a plurality of coordinate data sets to one set of received data. The storage unit is preferably a high capacity storage device such as a semiconductor memory and a hard disk drive. The storage unit preferably has a function as a cine-memory. The storage unit stored received data before the received data is converted to a video signal for display.

According to another aspect of the present invention, it is preferable that, in the ultrasound diagnosis apparatus, the reference image contains a body mark (body symbol) and a probe mark (probe symbol) displayed overlapping the body mark. According to another aspect of the present invention, it is preferable that, in the ultrasound diagnosis apparatus, the body mark is a three-dimensional bodymark and the probe mark is a three-dimensional probe mark. The three-dimensional body mark is a three-dimensional image schematically representing a portion of a living body. The three-dimensional probe mark is a three-dimensional image schematically representing the probe. As a method of generating the marks (body mark and probe mark), various methods can be employed such as, for example, a method in which one of a plurality of marks which are prepared in advance is selected, a method in which a mark is generated at a necessary time through software processing, or a combination of these methods. Alternatively, it is possible to employ a configuration in which the probe position is automatically identified and the probe orientation is manually set or a configuration in which the probe position is manually set and the probe orientation is automatically identified. Each mark may be either a monochrome image or a color image.

The body mark may be a two-dimensional image, in which case it may also be a line drawing. The probe mark may be represented as a model which simulates an approximate shape of the probe or may be a symbol such as an arrow or a box representing a position (and/or orientation) where the probe contacts. Alternatively, the probe mark may be a three-dimensional image which accurately represents the actual probe. It is possible to select a type of the probe mark based on the type of probe which is actually used.

According to another aspect of the present invention, it is preferable that, in the ultrasound diagnosis apparatus, the three-dimensional probe mark is displayed on the three-dimensional body mark at a position determined according to the coordinate data. According to another aspect of the present invention, it is preferable that, in the ultrasound diagnosis apparatus, the three-dimensional probe mark is displayed on the three-dimensional body mark with an orientation based on the coordinate data.

According to another aspect of the present invention, it is preferable that, in the ultrasound diagnosis apparatus, the storage unit further stores body mark type information which is information of type of body mark and probe mark type information which is information of type of probe mark, and the body mark type information and the probe mark type information are referred to during generation of the reference image and the reference image is generated based on this information. With this structure, the type of the body mark and the type of the probe mark are automatically identified during reading (replay) of received data and suitable body mark and probe mark can be generated (or selected). The body mark type may be selected by the user when the received data is obtained or may be automatically identified from diagnosis items. Similarly, the probe type may be selected by the user when the received data is obtained or may be automatically identified based on obtained probe type data. It is also possible to allow the user to select the body mark type and the probe mark type when the image is replayed, as necessary.

According to another aspect of the present invention, it is preferable that, in the ultrasound diagnosis apparatus, the coordinate measuring unit comprises a magnetic field generator provided at one of the probe and a predetermined fixed location, a magnetic sensor provided at another one of the probe and the predetermined fixed location, and a coordinate data calculator which calculates the coordinate data based on an output signal of the magnetic sensor. Generally, a magnetic sensor of a relatively small size is provided within the probe or external to the probe and a magnetic field generator having a relatively large size is provided at a predetermined fixed location near a bed.

According to another aspect of the present invention, it is preferable that the ultrasound diagnosis apparatus further comprises an image recording unit which records an image containing the living body image and the reference image. As the recording unit, it is possible to employ, for example, a system in which an image is recorded to an electronic recording medium such as VTR and CD-ROM, a system in which an image is recorded through photography, and a system in which an image is recorded through printing on paper.

It is preferable to execute calibration before coordinate measurement in order to define a coordinate system which reflects the size and orientation of the subject. In the calibration, an operation is performed to adjust the size and scale of the body mark to conform with the actual size and scale of the subject. As a result of this process, it is possible to generate a reference image which accurately reflects the position of the probe (actual measurement part) on the subject. In the calibration, a method is preferably used in which a center position on the transmission/reception surface of the probe is sequentially contacted with a plurality of parts on the subject for calibration which are set in advance, and the size of the subject is measured (alternatively, it is also possible to use a method as described in Japanese Patent Application No. 2002-218497 which is not made public at the time of filing of a Japanese patent application for the present invention).
(2) According to another aspect of the present invention, there is provided an ultrasound diagnosis apparatus comprising a transportable probe according to claim 9.

According to another aspect of the present invention, it is preferable that, in the ultrasound diagnosis apparatus, a sequence of received data is sequentially read from the cine-memory to display a sequence of living body images as an animation image, and a sequence of coordinate data corresponding to the sequence of the received data is sequentially read from the coordinate data table to display a sequence of reference images as an animation image along with the sequence of living body images.

In a preferred embodiment of the present invention which will be described below, it is possible to automatically display a reference image (body mark and probe mark) which schematically represents the actual past measurement conditions, when a still image or an animation image from the cine-memory is replayed. With this configuration, no complicated user operations are required. In addition, because the actual measurement condition when the living body image is obtained can be accurately re-created, it is possible to provide information useful for diagnosis of diseases from the living body image.

### BRIEF DESCRIPTION OF THE DRAWINGS

A preferred embodiment of the present invention will be described in detail based on the following figures, wherein:
Fig. 1 is a block diagram showing an overall structure of an ultrasound diagnosis apparatus according to a preferred embodiment of the present invention;
Fig. 2 is a diagram showing an example of a specific structure of a coordinate data table shown in Fig. 1;
Fig. 3 is a conceptual diagram for explaining a generation process of a reference image;
Fig. 4 is a diagram for explaining a coordinate system defined through calibration; and
Fig. 5 is a diagram for explaining simultaneous display of a reference image and a living body image.

### DESCRIPTION OF PREFERRED EMBODIMENT

A preferred embodiment (hereinafter referred to simply as "embodiment") of the present invention will now be described.

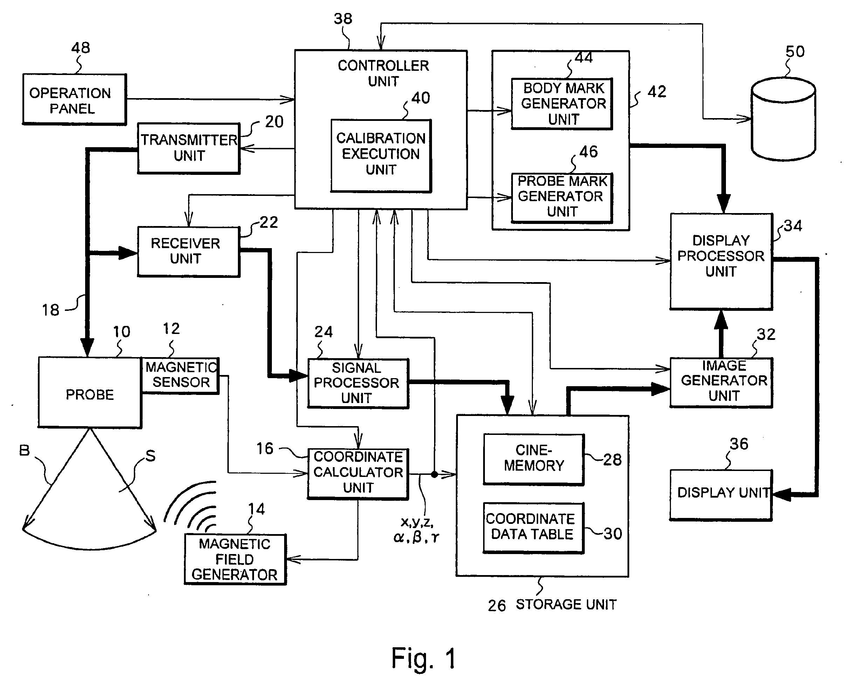

Fig. 1 is a block diagram showing an overall structure of an ultrasound diagnosis apparatus according to a preferred embodiment of the present invention.

A probe 10 is a transportable device for transmitting and receiving ultrasound. The probe 10 has a transducer array including a plurality of transducer elements in the structure exemplified in Fig. 1. The transducer array generates an ultrasound beam B. By electronically scanning with the ultrasound beam B, a two-dimensional scanning plane S is generated. As a method of electronic scanning, it is possible to employ, for example, an electronic sector scanning system or an electronic linear scanning system. It is also possible to provide a 2D (two-dimensional) transducer array in the probe 10 to form a 3D (three-dimensional) data obtaining space.

An ultrasound diagnosis apparatus according to the embodiment comprises, as means for measuring coordinates, a magnetic field generator 14, a magnetic sensor 12, and a coordinate calculator unit 16. In the configuration shown in Fig. 1, the magnetic field generator 14 is provided at a predetermined fixed location, such as a position near a bed (not shown) on which a patient is located. The magnetic sensor 12, on the other hand, is provided on the probe 10 in the example configuration of Fig. 1. More specifically, the magnetic sensor 12 is stored and located within a resin case in the probe 10. Various devices may be used as the magnetic field generator 14 and the magnetic sensor 12, as long as these devices can measure a three-dimensional position and a three-dimensional orientation of the probe 10. The magnetic field generator 14 has, for example, three magnetic field generator coils provided corresponding to three axes which are perpendicular to each other. These three coils are driven in a time divisional manner. The magnetic sensor 12 comprises, for example, three magnetic filed detector coils provided corresponding to three axes which are perpendicular to each other. The coordinate calculator unit 16 calculates a spatialposition (x, y, z) of the probe 10 anda rotational angle (α, β, γ) of the probe 10 with respect to the axes based on output signals of the coils output from the magnetic sensor 14. The coordinate measurement technique itself is a known technique. The definition of the components of the coordinate system may be other than those described above.

The probe 10 is connected to a main system of the apparatus through a cable 18. That is, the probe 10 in the embodiment is transportable and is, in general, used in contact with a surface of the body of the subject. Alternatively, it is also possible to use a probe 10 which is inserted into a body orifice, such the esophagus.

A structure of the main system of the apparatus will now be described. A transmitter unit 20 functions as a transmission beam former. The transmitter unit 20 supplies, to the plurality of transducer elements, a plurality of transmission signals to which a delay process is applied, under the control of a controller unit 38. A receiver unit 22 functions as a reception beam former. The receiver 22 applies a phase adjusting and summing process to a plurality of reception signals output from the plurality of transducer elements under a control of the controller unit 38.

A signal processor unit 24 applies processes such as detection and logarithmic compression to the phase adjusted and summed reception signal output from the receiver unit 22. These processes may alternatively be applied downstream of a storage unit 26 which will be described below. In this configuration, an RF signal is stored in the storage unit 26. The storage unit 26 stores reception signal (received data) before coordinates are converted. Alternatively, it is also possible to store, in the storage unit 26, received data after coordinates are converted.

In the embodiment, the storage unit 26 has a cine-memory 28 and a coordinate data table 30. The cine-memory 28 stores received data of a plurality of frames which are input in time series. The cine-memory 28 has a storage structure similar to a ring buffer. The cine-memory 28 always stores a sequence of received data from a most recent frame to a frame of a predetermined time before. As is known, when a user applies a freeze operation, transmission and reception of the ultrasound is terminated. At this point, the stored content in the cine-memory 28 is frozen. When an ultrasound image is to be displayed in real time, it is possible to employ a configuration in which received data output from the signal processor unit 24 is temporarily stored in the cine-memory 28 and the received data is immediately read from the cine-memory 28. Alternatively, it is also possible to output the received data output from the signal processor unit 24 directly to an image generator unit 32 which will be described later and, at the same time, store the received data in the cine-memory 28.

The coordinate data table 30 is a table which stores a plurality of coordinate data correlated to a plurality of received data stored in the cine-memory 28. When certain received data is stored in the cine-memory 28, coordinate data correlated to the received data is stored in the coordinate data table 30. The coordinate data represents a position and an orientation of the probe 10 at the time when the received data is obtained. In the present embodiment, one item of coordinate data is correlated to and stored with one item of received data. Therefore, similar to the cine-memory 28, the coordinate data table 30 also has a storage structure similar to a ring buffer.

The management unit of the received data in the cine-memory 28 may be, for example, beams, frames, or volumes. The management unit of coordinate data in the coordinate data table 30 may also be a unit such as beams, frames, or volumes, similar to the management unit of the received data. In the present embodiment, the correlation between the received data and the coordinate data is managed with one frame composed of a plurality of beams as the management unit, Alternatively, in this configuration, one coordinate data may be correlated to a plurality of received data. Alternatively, a plurality of coordinate data may be correlated to one received data. In the present embodiment, the coordinate data is formed as a set of parameter values of x, y, z, α, β, and γ, as already described above. Among these parameters, measurement and storage of, for example, known values or constant values may be omitted. Alternatively, it is also possible to form the coordinate data with only parameter values, among the six parameter values, necessary for generation of the reference image. In any case, because the coordinate data is correlated to and stored with the received data, it is possible to use the coordinate data correlated to the received data when the received data is replayed, as will be described later. In other words, the present embodiment has an advantage that the body mark and the probe mark can be automatically generated and displayed using the coordinate data. Control to write data and control to read data to and from the storage unit 26 are executed by the controller unit 38 which will be described later. It is also possible to store an electrocardiographic signal in the cine-memory 28 along with the received data.

The image generator unit 32 is means for generating an ultrasound image as a living body image based on the received data and has, for example, a digital scan converter (DSC). In the present embodiment, a two-dimensional ultrasound image (image of tissue and image of blood stream, etc.) are generated. Alternatively, a three-dimensional image may be generated or an M mode image or a Doppler waveform image may be generated.

A display processor unit 34 synthesizes image data as living body image output from the image generator unit 32 and graphical data output from a graphics generator unit 42 which will be described below and outputs data which represents a synthesized image. The image data output from the display processor unit 34 is sent to a display unit 36. A synthesized image including the living body image and the graphical image is displayed on a screen of a display unit 36.

The display unit 36 may alternatively be formed with two display devices (main display device and auxiliary display device). In this configuration, the living body image may be displayed on one of the two display devices and the graphical image may be displayed on the other of the two display devices. The synthesized image can be recorded on a recording medium such as a VTR or CD-ROM, printed on paper, or captured as a photograph. Because the synthesized image contains the reference image, it is possible to record the reference image along with the living body image.

The controller unit 38 has a CPU for executing software instructions. The controller unit 38 controls operations of the structures shown in Fig. 1 and, in particular, supplies a graphics generation condition to the graphics generator unit 42 which is substantially formed by a software.

The controller unit 38 has a calibration function, here embodied within a calibration execution unit 40. A calibration process is executed before measurement in order to correlate (conform) a scale or a size in the body mark to a real scale or a real size in the subject by identifying a coordinate system in the subject.

A specific example of calibration will now be described. In the present embodiment, with an operation by the user, a center position of a transmission/reception surface of the probe 10 is contacted to a plurality of specific positions for calibration defined on the subject and coordinate data of the probe is obtained at each of the specific positions. A coordinate system in the subj ect is then identified based on the plurality of coordinate data corresponding to the plurality of specific positions. According to this identification, it is possible to conform the coordinate system with respect to the body mark to the coordinate system with respect to the subject. The conforming of coordinate systems includes matching of origins, matching of the scales or sizes, etc. When mismatch of coordinate systems between the subject and the body mark does not pose a problem, it is not necessary to apply the calibration process.

When the calibration process as described above is executed, a result of the calibration is supplied from the controller unit 38 to the coordinate calculator unit 16. In an ultrasound diagnosis after the calibration, the coordinate calculator unit 16 calculates the probe coordinates based on an output signal of the magnetic sensor 12 and according to a coordinate system based on the subject defined through the calibration. The coordinate calculator unit 16 outputs the coordinate data, which is the result of the calculation, to the coordinate data table 30 of the storage unit 26 and also to the controller unit 38. The controller unit 38 receives the coordinate data output from the coordinate calculator unit 16 when the ultrasound image is to be displayed in real time. When, on the other hand, an image is to be replayed using the cine-memory 28, the controller unit 38 receives coordinate data read from the coordinate data table 30. The controller unit 38 controls generation of the body mark and generation of the probe mark based on the received coordinate data, as will be described below. In the present embodiment, the three-dimensional body mark and the three-dimensional probe mark can be automatically displayed both in a configuration in which the ultrasound image is to be displayed in real time (real time display mode) and in a configuration in which the ultrasound image is to be replayed and displayed using received data which is stored in the cine-memory functioning as a storage device (replay display mode).

In the example of the present embodiment, the graphics generator unit 42 comprises a body mark generator unit 44 and a probe mark generator unit 46. These generator units 44 and 46 are substantially realized by software in the present embodiment. In the generator units 44 and 46, a mark corresponding to the condition output by the controller unit 38 is selected from among a plurality of marks which are provided in advance or a mark is generated based on the condition output by the controller unit 38 when the controller unit 38 outputs the condition. In the present embodiment, the body mark generator unit 44 generates a monochrome or color three-dimensional body mark and the probe mark generator unit 46 generates a monochrome or color three-dimensional probe mark. The body mark and the probe mark may alternatively be digital images captured by a digital camera.

In the present embodiment, the graphics generator unit 42 functions both in the real time display mode and replay display mode. In other words, in both display modes, the body mark and the probe mark can be automatically generated according to a display condition output from the controller unit 38. Graphical data containing these marks is supplied to the display processor unit 34. The display processor unit 34 executes a process to synthesize the living body image data and the graphical data and supplies the data of the synthesized image generated in this process to the display unit 36.

More specifically, the body mark generator unit 44 can generate a plurality of types of body marks. More specifically, the body mark generator unit 44 can generate a three-dimensional body mark having a suitable form corresponding to the diagnosis item, diagnosis part, type of patient, and size of patient. Information indicating the type of body mark is stored in the coordinate data table 30 as will be described below. The probe mark generator unit 46, on the other hand, can generate a plurality of types of probe marks. More specifically, the probe mark generator unit 46 can generate a three-dimensional probe mark having a shape corresponding to the type of probe. Information indicating the type of the probe mark is stored in the coordinate data table 30 as will be described later. It is also possible to allow the direction for displaying the body mark (direction of view line) to be variable. The position and orientation of the probe mark is adaptively set based on the actual position and orientation of the probe. On the display screen, the probe mark is displayed overlapping the body mark. In this manner, the actual usage state of the probe is simulated and re-created on the display screen. In order to automatically generate a three-dimensional mark, it is possible to employ a known three-dimensional image constructing method such as, for example, volume rendering and surfacing method.

An external storage device 50 is connected to the controller unit 38 and stores various data necessary for control of operations by the controller unit 38. In addition, an operation panel 48 is connected to the controller unit 38. A user can set and input various parameters using the operation panel 48.

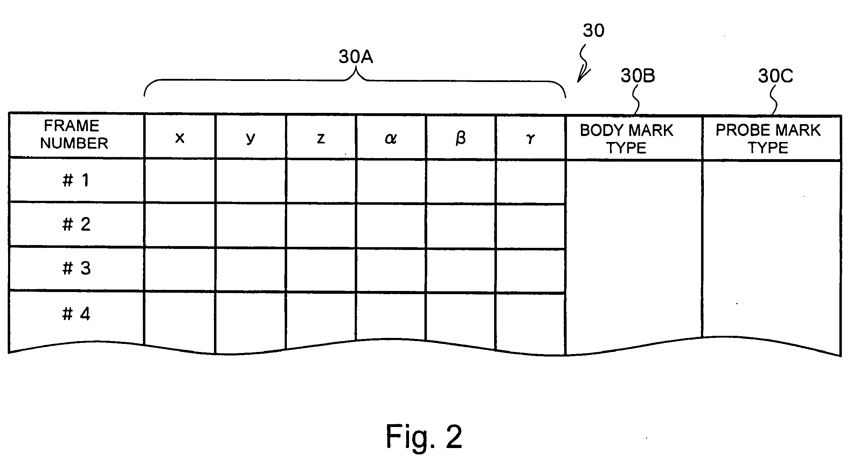

Fig. 2 shows a specific example structure of the coordinate data table 30 shown in Fig. 1. In the structure exemplified in Fig. 2, the received data is managed in units of frames. Specific coordinate data 30A is correlated to the frame number. The coordinate data is made of data x, y, and z representing the spatial position of the probe and data α, β, and γ representing the orientation of the probe. This configuration, however, is only exemplary, and coordinate data of various forms may be used as long as the coordinate data allows appropriate display of the marks.

In the present embodiment, the coordinate data table 30 stores body mark type information 30B and probe mark type information 30C in addition to the coordinate data. The type of the body mark is automatically selected based on medical information and patient information or is selected by the user. The type of the probe mark is automatically identified or is registered by the user. Because the information 30B and 30C are stored in the coordinate data table, the types of marks can be automatically selected using the information 30B and 30C both in the real time display mode and in the replay display mode. Alternatively, it is also possible to employ a configuration in which the user designates one or both of the body mark type and the probe mark type after the freeze operation as necessary.

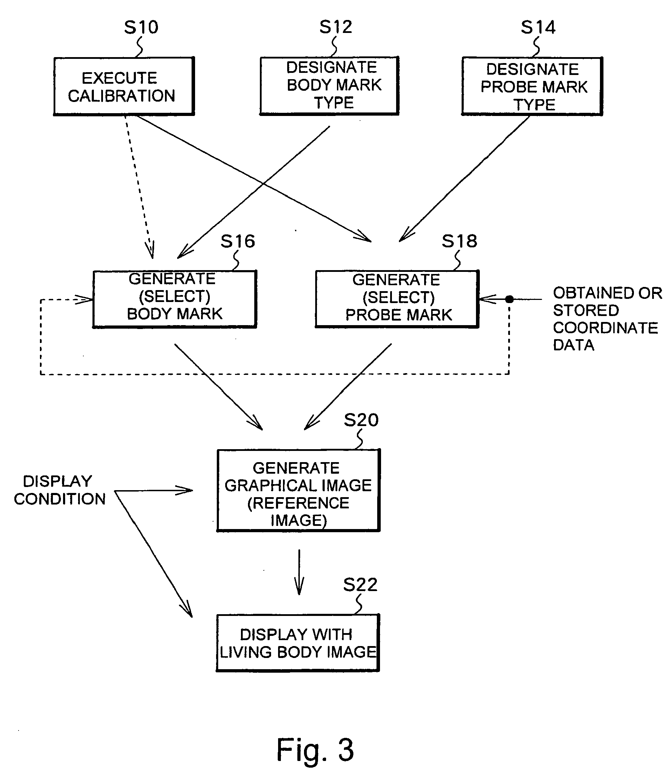

Fig. 3 is a conceptual diagram showing a process for generating a reference image. In step S10, calibration as described above is executed. Either prior to or following the calibration, in step S12, a body mark type is designated and, in step S14, a probe mark type is designated. The mark types are designated automatically or by the user. In step S16, a body mark is generated and, in step S18, a probe mark is generated. With this configuration, in a real time display mode, coordinate data which is currently obtained is used and a probe mark is displayed at an appropriate position with an appropriate orientation on the body mark. In other words, a situation reflecting the actual measurement state is schematically re-created. This is similar in the replay display mode using the cine-memory. That is, in the replay display mode, coordinate data stored in the coordinate data table is read and a probe mark is displayed at an appropriate position with an appropriate orientation on the body mark based on the read coordinate data.

In the above-described step S18, the probe mark is generated according to a probe mark type designated in step S14. In this process, a result of execution of the calibration is considered. Similarly, in the above-described step S16, the body mark is generated based on the body mark type designated in step S12. In this process, a result of execution of the calibration is considered and the coordinate data is considered as necessary. For example, a specific body mark is selected from among a plurality of body marks belonging to the designated body mark type, according to the coordinate data.

In step S20, a graphical image (reference image) is generated by synthesizing the body mark (graphical data) and the probe mark (graphical data). More specifically, the reference image is generated according to the display condition. For example, a color coding process of skin color may be applied to the body mark and a color coding process reflecting the actual color of the probe may be applied to the probe mark. In step S22, a graphical image (that is, a reference image) is displayed on the screen along with the living body image according to the display condition which is set by the controller unit.

The above-described processes are executed for each frame. For example, when the received data from the cine-memory is to be displayed as an animation image, the movement of the probe when the received data is obtained is re-created as the movement of the probe mark.

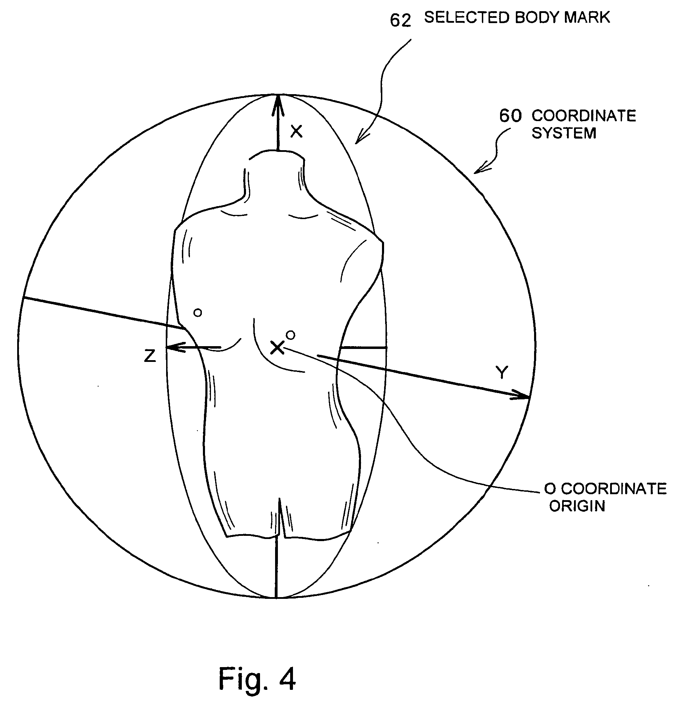

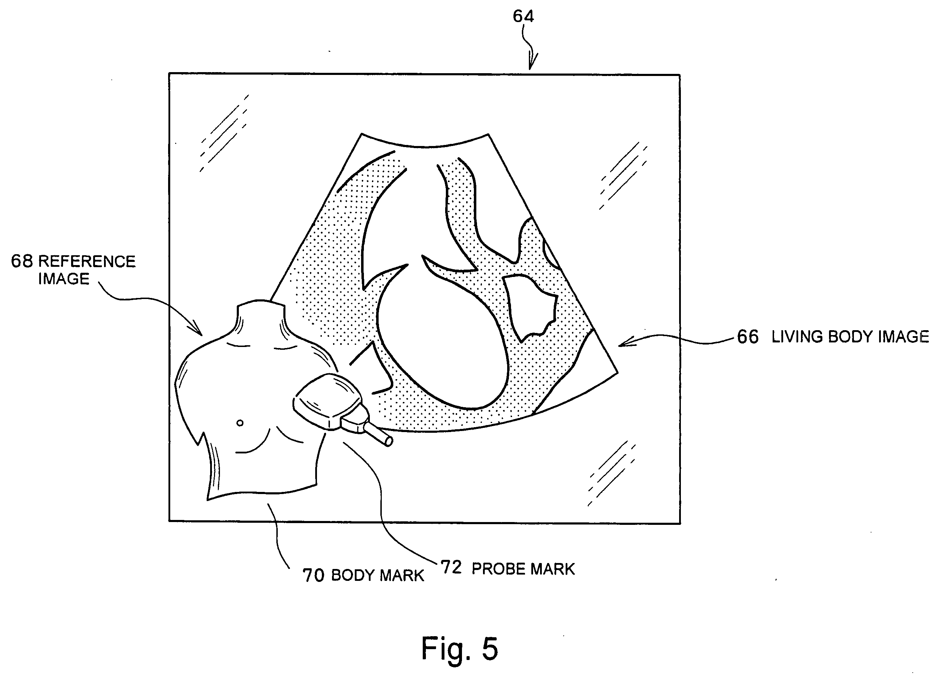

Fig. 4 shows a coordinate system 60 defined regarding the body mark (or the subject). The coordinate system 60 is defined in the calibration process described above. Fig. 4 shows a typical body mark 62. The coordinate system 60 has three perpendicular axes X, Y, and Z which pass though a coordinate origin O. The position and orientation of the probe in such coordinate system 60 is measured in real time by the above-described coordinate measuring means. The probe mark is displayed on the body mark according to the coordinate data obtained through the measurement, as shown in Fig. 5.

Fig. 5 shows an example of a display screen 64. A living body image 66 and a reference image 68 are shown on the display screen 64. As described above, the reference image 68 includes a body mark 70 and a probe mark 72. These marks are three-dimensional images having a perceived depth. When the probe is moved on the surface of the living body in the real time display mode, the probe mark 72 on the body mark 70 also moves accordingly. This is similar in the replay display mode. In the related art, selection operation and positioning operation by the user is necessary for displaying the body mark 70 and the probe mark 72 in the replay display mode. With the present embodiment, however, such special operations are not necessary. More specifically, because the coordinate data is stored correlated to the received data, it is possible to read and use the coordinate data correlated to the received data when the received data is read. As a result, it is possible to automatically display the probe mark 72 at an appropriate position with an appropriate orientation on the body mark 70. With this structure, it is possible to schematically re-create the situation of the ultrasound diagnosis as the data is actually obtained. In particular, in the present embodiment, because the three-dimensional body mark and the three-dimensional probe mark are displayed, it is possible to provide a display which allows intuitive recognition of the body part being imaged and the direction from which it is being imaged.

A display position of the reference image 68 on the display screen 64 can be arbitrarily set by the user. It is desirable to allow the user to arbitrarily set the size of the reference image. Alternatively, it is also possible to prepare a plurality of body marks representing the same part and having different directions and to automatically select the body mark to be displayed according to the position of the probe. It is also possible to allow generation of a plurality of body marks which can represents the state of a patient lying on a bed.

## Claims

1. An ultrasound diagnosis apparatus comprising:
a probe (10) which transmits and receives ultrasound and outputs received data;
a coordinate measuring unit which measures at least one of a spatial position and orientation of the probe (10) and outputs coordinate data (30A) representing a result of measurement;
a storage unit (26) which includes a received data memory (28) which stores the received data and a coordinate data memory (30) which stores coordinate data (30A) correlated to the received data;
a read controller unit (38) which reads from the received data memory (26) the received data, and reads from the coordinate data memory (30) the coordinate data (30A) correlated to the read received data in a replay display mode;
a living body image generator unit (32) which generates a living body image (66) based on the received data;
a reference image generator unit which generates a reference image (68) based on the coordinate data (30A); and
a display unit (36) which displays the living body image (66) and the reference image (68)
***characterized in***
- **that** the storage unit (26) further stores mark type information which comprises body mark type information (30B) which is information regarding body mark type and probe mark type information (30C) which is information regarding probe mark type,
- wherein the body mark type information (30B) and the probe mark type information (30C) are referred to during generation of the reference image (68) and the reference image (68) is generated based on these pieces of information,
- wherein the living body image generator unit (32) generates the living body image based on the read received data and
- wherein the reference image generator generates the reference image (68) based on the read coordinate data (30A) and the mark type information, and
- the reference image (68) contains a body mark (70) and a probe mark (72),
- **that** the received data memory is a cine-memory having a ring buffer structure, and
- **that** the coordinate data memory is a coordinate data table having a ring buffer structure.

2. An ultrasound diagnosis apparatus according to Claim 1, wherein
the probe mark (72) is displayed overlapping the body mark (70).

3. An ultrasound diagnosis apparatus according to Claim 2, wherein
the body mark (70) is a three-dimensional body mark, and
the probe mark (72) is a three-dimensional probe mark.

4. An ultrasound diagnosis apparatus according to Claim 3, wherein
the three-dimensional probe mark (72) is displayed on the three-dimensional body mark at a position determined according to the coordinate data (30A).

5. An ultrasound diagnosis apparatus according to Claim 4, wherein
the three-dimensional probe mark (72) is displayed on the three-dimensional body mark (70) with an orientation determined according to the coordinate data (30A).

6. An ultrasound diagnosis apparatus according to Claim 1, wherein
the body mark type information (30B) and the probe mark type information (30C) are referred to during generation of the reference image (68) and the reference image (68) is generated based on these information.

7. An ultrasound diagnosis apparatus according to Claim 1, wherein
the coordinate measuring unit comprises:
a magnetic field generator (14) provided at one of either the probe (10) or a predetermined fixed location;
a magnetic sensor (12) provided at the other one of the probe (10) or the predetermined fixed location; and
a coordinate data calculator (16) which calculates the coordinate data based on an output signal of the magnetic sensor (12).

8. An ultrasound diagnosis apparatus according to Claim 1, further comprising:
an image recording unit which records an image containing the living body image (66) and the reference image (68).

9. An ultrasound diagnosis apparatus according to claim 1, wherein
the probe is a transportable probe (10) which outputs received data of frames by contacting a subject and repeatedly scanning with an ultrasound beam;
the coordinate measuring unit comprising a magnetic sensor (12) provided on the probe (10) and a magnetic field generator (14) adapted to be provided at a predetermined fixed location near the subject, wherein the coordinate measuring unit measures a spatial position and orientation of the probe (10) in real time and outputs coordinate data representing a result of the measurement.

10. An ultrasound diagnosis apparatus according to Claim 9, wherein
a sequence of received data is sequentially read from the cine-memory (28) to display a sequence of living images (66) as an animation image, and
a sequence of coordinate data (30A) corresponding to the sequence of the received data is sequentially read from the coordinate data table (30) to display a sequence of reference images (68) as an animation image along with the sequence of living body images (66).

## Patentansprüche

1. Ultraschalldiagnosegerät mit:
einer Sonde (10), die Ultraschall aussendet und empfängt und empfangene Daten ausgibt;
einer Koordinatenmesseinheit, die mindestens eines von einer räumlichen Position und Orientierung der Sonde (10) misst und Koordinatendaten (30A) ausgibt, die ein Messergebnis darstellen;
einer Speichereinheit (26), die einen Speicher (28) für empfangene Daten, der die empfangenen Daten speichert, und einen Speicher (30) für Koordinatendaten aufweist, der Koordinatendaten (30A) speichert, die mit den empfangenen Daten korreliert sind;
einer Auslesesteuerungseinheit (38), die in einem Wiedergabeanzeigemodus von dem Speicher (26) für empfangene Daten die empfangenen Daten ausliest und von dem Speicher (30) für Koordinatendaten die Koordinatendaten (30A) ausliest, die mit den ausgelesenen empfangenen Daten korreliert sind;
einer Bilderzeugungseinheit (32) für einen lebenden Körper, die ein Bild (66) eines lebenden Körpers erzeugt, basierend auf den empfangenen Daten;
einer Referenzbilderzeugungseinheit, die ein Referenzbild (68) basierend auf den Koordinatendaten (30A) erzeugt; und
einer Anzeigeeinheit (36), die das Bild (66) des lebenden Körpers und das Referenzbild (68) anzeigt,
**dadurch gekennzeichnet,**
- **dass** die Speichereinheit (26) ferner zeichenartige Information speichert, die zeichenartige Information (30B) des Körpers, welche Information bezüglich eines zeichenartigen Körpers ist und zeichenartige Information (30C) der Sonde aufweist, welche Information bezüglich einer zeichenartigen Sonde ist,
- wobei während dem Erzeugen des Referenzbilds (68) auf die zeichenartige Information (30B) des Körpers und die zeichenartige Information (30C) der Sonde zurückgegriffen wird und das Referenzbild (68) basierend auf diesen Informationsteilen erzeugt wird,
- wobei die Bilderzeugungseinheit (32) für einen lebenden Körper das Bild des lebenden Körpers basierend auf den ausgelesenen empfangenen Daten erzeugt und
- wobei der Referenzbilderzeuger das Referenzbild (68) basierend auf den ausgelesenen Koordinatendaten (30A) und der zeichenartigen Information erzeugt, und
- das Referenzbild (68) ein Körperzeichen (70) und ein Sondenzeichen (72) umfasst,
- **dass** der Speicher für empfangene Daten ein Cine-Speicher ist, der eine Ringpufferstruktur hat, und
- **dass** der Speicher für Koordinatendaten eine Koordinatendatentabelle ist, die eine Ringpufferstruktur hat.

2. Ultraschalldiagnosegerät nach Anspruch 1,
wobei das Sondenzeichen überlappend mit dem Körperzeichen (70) angezeigt wird.

3. Ultraschalldiagnosegerät nach Anspruch 2,
wobei das Körperzeichen (70) ein dreidimensionales Körperzeichen ist und das Sondenzeichen (72) ein dreidimensionales Sondenzeichen ist.

4. Ultraschalldiagnosegerät nach Anspruch 3,
wobei das dreidimensionale Sondenzeichen (72) auf dem dreidimensionalen Körperzeichen an einer Position angezeigt wird, die gemäß den Koordinatendaten (30A) bestimmt wird.

5. Ultraschalldiagnosegerät nach Anspruch 4,
wobei das dreidimensionale Sondenzeichen (72) auf dem dreidimensionalen Körperzeichen (70) mit einer Orientierung angezeigt wird, die gemäß den Koordinatendaten (30A) bestimmt wird.

6. Ultraschalldiagnosegerät nach Anspruch 1,
wobei während dem Erzeugen des Referenzbilds (68) auf die zeichenartige Information (30B) des Körpers und die zeichenartige Information (30C) der Sonde Bezug genommen wird und das Referenzbild (68) basierend auf diesen Informationen erzeugt wird.

7. Ultraschalldiagnosegerät nach Anspruch 1,
wobei die Koordinatenmesseinheit aufweist:
einen Magnetfelderzeuger (14), der an einem von entweder der Sonde (10) oder
einem vorbestimmten festen Ort vorgesehen ist;
einen magnetischen Sensor (12), der an dem anderen von der Sonde (10) oder
dem vorbestimmten festen Ort vorgesehen ist; und
einen Koordinatendatenrechner (16), der die Koordinatendaten basierend auf einem Ausgabesignal des magnetischen Sensors (12) berechnet.

8. Ultraschalldiagnosegerät nach Anspruch 1,
das ferner aufweist:
eine Bildaufnahmeeinheit, die ein Bild aufnimmt, welches das Bild (66) des lebenden Körpers und das Referenzbild (68) umfasst.

9. Ultraschalldiagnosegerät nach Anspruch 1,
wobei die Sonde eine transportable Sonde (10) ist, die empfangene Daten von Einzelbildern durch Berühren einer Zielperson und wiederholtem Scannen mit einem Ultraschallstrahl ausgibt;
wobei die Koordinatenmesseinheit einen magnetischen Sensor (12) aufweist, der auf der Sonde (10) vorgesehen ist, und einen Magnetfelderzeuger (14) aufweist, der ausgebildet ist, um an einem vorbestimmten festen Ort nahe der Zielperson vorgesehen zu sein, wobei die Koordinatenmesseinheit eine räumliche Position und Orientierung der Sonde (10) in Echtzeit misst und Koordinatendaten ausgibt, die ein Ergebnis der Messung darstellen.

10. Ultraschalldiagnosegerät nach Anspruch 9,
wobei eine Sequenz von empfangenen Daten sequenziell von dem Cine-Speicher (28) ausgelesen wird, um eine Sequenz von lebenden Bildern (66) als ein animiertes Bild anzuzeigen, und
wobei eine Sequenz von Koordinatendaten (30A), die der Sequenz von empfangenen Daten entspricht, sequenziell von der Koordinatendatentabelle (30) ausgelesen wird, um eine Sequenz von Referenzbildern (68) als ein animiertes Bild zusammen mit der Sequenz von Bildern (66) des lebenden Körpers anzuzeigen.

## Revendications

1. Un appareil de diagnostic à ultrasons comprenant:
une sonde (10) qui transmet et reçoit des ultrasons et sort des données reçues,
une unité de mesure de coordonnées qui mesure au moins une d'une position et
d'une orientation spatiales de la sonde (10) et sort des données de coordonnées (30A) représentant un résultat de mesure,
une unité de stockage (26) qui inclut une mémoire de données reçues (28) qui stocke les données reçues et une mémoire de données de coordonnées (30) qui stocke des données de coordonnées (30A) corrélées aux données reçues,
une unité de contrôle de lecture (38) qui fait une lecture, à partir de la mémoire de données reçues (26), des données reçues et qui fait une lecture, à partir de la mémoire de données de coordonnées (30), les données de coordonnées (30A) corrélées aux données reçues de lecture dans un mode d'affichage reproduction,
une unité productrice d'image de corps vivant (32) qui produit une image de corps vivant (66) sur la base des données reçues,
une unité productrice d'image de référence qui produit une image de référence (68) sur la base des données de coordonnées (30A), et
une unité d'affichage (36) qui affiche l'image de corps vivant (66) et l'image de référence (68)
**caractérisé en ce que**
- l'unité de stockage (26) stocke en outre des informations au type de marque qui comprennent des informations relatives au type de marque de corps (30B), qui sont des informations concernant un type de marque de corps, et des informations au type de marque de sonde (30C), qui sont des informations concernant un type de marque de sonde,
- tandis les informations au type de marque de corps (30B) et les informations au type de marque de sonde (30C) servent de fondement au cours de la production de l'image de référence (68) et l'image de référence (68) est produite sur la base de ces éléments d'information,
- tandis que l'unité productrice d'image de corps vivant (32) produit l'image de corps vivant sur la base des données reçues de lecture et
- tandis que le producteur d'image de référence produit l'image de référence (68) sur la base des données de coordonnées de lecture (30A) et les informations au type de marque, et
- l'image de référence (68) contient une marque de corps (70) et une marque de sonde (72),
- que la mémoire de données reçues est une cine-mémoire ayant une structure de tampon circulaire, et
- que la mémoire de données de coordonnées est un tableau de données de coordonnées ayant une structure de tampon circulaire.

2. L' appareil de diagnostic à ultrasons selon la revendication 1,
tandis que la marque de sonde (72) est affichée superposant la marque de corps (70).

3. L' appareil de diagnostic à ultrasons selon la revendication 2,
tandis que la marque de corps (70) est une marque de corps tridimensionnelle et la marque de sonde (72) est une marque de sonde tridimensionnelle.

4. L' appareil de diagnostic à ultrasons selon la revendication 3,
tandis que la marque de sonde tridimensionnelle (72) est affichée sur la marque de corps tridimensionnelle à un emplacement déterminé selon les données de coordonnées (30A).

5. L'appareil de diagnostic à ultrasons selon la revendication 4,
tandis que la marque de sonde tridimensionnelle (72) est affichée sur la marque de corps tridimensionnelle (70) avec une orientation déterminée selon les données de coordonnées (30A).

6. L'appareil de diagnostic à ultrasons selon la revendication 1,
tandis que les informations relatives au type de marque de corps (30B) et les informations au type de marque de sonde (30C) servent de fondement au cours de la production de l'image de référence (68) et l'image de référence (68) est produite sur la base de ces informations.

7. L' appareil de diagnostic à ultrasons selon la revendication 1,
tandis que l'unité de mesure de coordonnées comprend:
un producteur de champ magnétique (14) fourni soit au niveau de la sonde (10),
soit au niveau d'un emplacement fixe prédéterminé,
un capteur magnétique (12) fourni soit au niveau de la sonde (10), soit au niveau d'un emplacement fixé prédéterminé, et
un calculateur de données de coordonnées (16) qui calcule les données de coordonnées sur la base d'un signal de sortie du capteur magnétique (12).

8. L' appareil de diagnostic à ultrasons selon la revendication 1 comprenant en outre:
une unité d'enregistrement d'image qui enregistre une image contenant une image de corps vivant (66) et l'image de référence (68).

9. L' appareil de diagnostic à ultrasons selon la revendication 1,
tandis que la sonde est une sonde transportable (10) qui produit des données reçues de cadres en contactant un sujet et en scannant à plusieurs reprises par le biais d'un faisceau d'ultrasons,
l'unité de mesure de coordonnées comprenant un capteur magnétique (12) fournie sur la sonde (10) et un producteur de champ magnétique (14) adapté pour être fourni à un emplacement fixe prédéterminé près du sujet, tandis que l'unité de mesure de coordonnées mesure un emplacement et une orientation spatiales de la sonde (10) en temps réel et sort des données de coordonnées représentant un résultat de la mesure.

10. L'appareil de diagnostic à ultrasons selon la revendication 9,
tandis qu'une séquence de données reçues est lue séquentiellement à partir de la cine-mémoire (28) pour afficher une séquence d'images vivantes (66) en tant qu'image d'animation, et
une séquence de données coordonnées (30A) correspondant à la séquence des données reçues est lue séquentiellement à partir du tableau de données de coordonnées (30) pour afficher une séquence d'images de référence (68) en tant qu'image d'animation avec la séquence d'images de corps vivant (66).
